(19) Europäisches Patentamt / European Patent Office / Office européen des brevets

(11) **EP 2 512 343 B1**

(12) **EUROPEAN PATENT SPECIFICATION**

(45) Date of publication and mention of the grant of the patent:
**16.03.2016 Bulletin 2016/11**

(51) Int Cl.:
**A61B 6/00** *(2006.01)*    **G06T 7/00** *(2006.01)*
**G06T 7/20** *(2006.01)*    **H04N 5/325** *(2006.01)*

(21) Application number: **10837680.7**

(22) Date of filing: **10.12.2010**

(86) International application number:
**PCT/JP2010/072730**

(87) International publication number:
**WO 2011/074655 (23.06.2011 Gazette 2011/25)**

(54) **X-RAY IMAGE PROCESSING APPARATUS, X-RAY IMAGE PROCESSING METHOD, AND STORAGE MEDIUM FOR COMPUTER PROGRAM**

RÖNTGENBILDVERARBEITUNGSVORRICHTUNG, RÖNTGENBILDVERARBEITUNGSVERFAHREN UND SPEICHERMEDIUM FÜR EIN COMPUTERPROGRAMM

APPAREIL ET PROCEDE DE TRAITEMENT D'IMAGE PAR RAYONS X, ET SUPPORT DE STOCKAGE POUR PROGRAMME INFORMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2009 JP 2009288466**

(43) Date of publication of application:
**24.10.2012 Bulletin 2012/43**

(73) Proprietor: **Canon Kabushiki Kaisha Tokyo 146-8501 (JP)**

(72) Inventor: **MIYAMOTO, Hideaki Tokyo 146-8501 (JP)**

(74) Representative: **Williamson, Brian Canon Europe Ltd European Patent Department 3 Square, Stockley Park Uxbridge, Middlesex UB11 1ET (GB)**

(56) References cited:
JP-A- 5 167 927    JP-A- 8 336 519
JP-A- 61 199 389    JP-A- 2004 112 469
JP-A- 2007 215 930    JP-B2- 4 030 786
US-A- 5 285 786

- **PHILIPPE GAILLOUD ET AL: "Three-Dimensional Fusion Digital Subtraction Angiography: New Reconstruction Algorithm for Simultaneous Three-Dimensional Rendering of Osseous and Vascular Information Obtained during Rotational Angiography", AM J NEURORADIOL, vol. 26, 1 April 2005 (2005-04-01), pages 908-911, XP055074246,**

EP 2 512 343 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Technical Field

The present invention relates to a technique that obtains a contrast-agent injection region from an image that is obtained by digital subtraction angiography.

## Background Art

Since digital techniques have progressed in recent years, in many cases, digital processing is performed for images even in medical fields. Instead of conventional radiography using a film for X-ray diagnosis, a two-dimensional X-ray sensor that outputs an X-ray image as a digital image is being widely used. Digital image processing, such as gradation processing, for the digital image output by the two-dimensional X-ray sensor, is becoming more important.

An example of the digital image processing is DSA processing for acquiring digital subtraction angiography (hereinafter, referred to as "DSA image"). A DSA image is obtained such that images are acquired before and after a contrast agent is injected into an object, and the image before the contrast agent is injected (hereinafter, referred to as "mask image") is subtracted from the image after the contrast agent is injected (hereinafter, referred to as "live image"). In the subtraction processing of the mask image from the live image, a blood vessel region, which is a region of interest for the diagnosis, is held as a change region between the images caused by the injection of the contrast agent, and the other unnecessary region is eliminated as a background region to obtain a uniform region. Thus, the generated DSA image is useful for the diagnosis.

The purpose of use of the DSA image, in view of the diagnosis, is clear delineation of a blood vessel image with a contrast agent injected. The subtraction image obtained by subtracting the mask image from the live image has attained that purpose. To obtain further clear delineation, a contrast-agent injection region is separated from a background region that is other than the contrast-agent injection region by image analysis, and image-quality increase processing is performed by applying different image processing to these regions. The image-quality increase processing may be, for example, enhancement selectively for the contrast-agent injection region, noise reduction selectively for the background region, and generation of a road map image in which the contrast-agent injection region is superposed on the live image.

PTL 1 discloses a technique that performs threshold processing for comparing each portion in a subtraction with a predetermined value in order to reliably distinguish a blood vessel region from the other region in the subtraction image, and separates the blood vessel region, which is a region of interest, from the other region, on the basis of the result so that only the blood vessel region is displayed in an enhanced manner. PTL 2 discloses a technique that obtains a gradient value and a gradient line toward a blood vessel from a horizontal pixel-value gradient and a vertical pixel-value gradient for each pixel in a subtraction image. A profile is generated for the gradient value on the gradient line or a pixel value. Then, a local maximum point or a global maximum point is extracted as a contour point or a core line point.

Meanwhile, when the image-quality increase processing is used, the contrast-agent injection region has to be separated from the background region that is other than the contrast-agent injection region. If motion of the object does not appear between the mask image and live image, or under an ideal condition in which the motion of the object is completely corrected, the separation processing can be easily performed by the analysis of the subtraction image.

However, in general, the motion of the object appears between the mask image and the live image, and it is difficult to correct the movement. Hence, a motion artifact may appear in the region other than the contrast-agent injection region in the DSA image. Owing to this, with the conventional methods, an edge generated by the motion artifact resulted from the motion of the object may be detected. Also, with the conventional methods, a region with a large difference between pixel values, such as the boundary between the object and a transparent region, or the boundary between a lung field region or a metal region and the other region, is extracted as a high pixel-value region in the DSA image.

## Citation List

## Patent Literature

PTL 1 Japanese Patent Publication No. 04-030786
PTL 2 Japanese Patent Laid-Open No. 05-167927

PHILIPPE GAILLOUD ET AL: "Three-Dimensional Fusion Digital Subtraction Angiography: New Reconstruction Algorithm for Simultaneous Three-Dimensional Rendering of Osseous and Vascular Information Obtained during Rotational Angiography", AM J NEURORADIOL, vol. 26, 1 April 2005 (2005-04-01), pages 908-911, XP055074246, discloses a 3D fusion digital subtraction angiography (FDSA), a new algorithm for rotational angiography that combines reconstructions of the blood vessels and the osseous from in a single 3D representation. 3D-FDSA is based on separate reconstructions of the mask and contrast sequences of the rotational acquisition. The two independent 3D data sets (3D-bone and 3D-digital subtraction angiography [DSA]) are fused in a single 3D representation. The algorithm uses a modification of the Feldkamp method that compensates for single intensity inhomogeneity inherent to the reconstruction of non-subtracted rotation acquisitions. By separately reconstructing the osseous and vascular information obtained from the rotational angiogram, 3D-FSDA provides optimal angiographic resolution and precise topographic analysis even when the studied vascular tree is in the immediate vicinity of bone.

## Summary of Invention

The present invention according to this application addresses the above problem, and provides a technique that accurately extracts a contrast-agent injection region.

The present invention is made in light of the situations. According to an aspect of the present invention, there is an X-ray image processing apparatus as claimed in claim 1.

Other features and advantages of the present invention will be apparent from the following description taken in conjunction with the accompanying drawings, in which like reference characters designate the same or similar parts throughout the figures thereof.

## Brief Description of Drawings

The accompanying drawings, which are incorporated in and constitute a part of the specification, illustrate embodiments of the invention, and together with the description, serve to explain the principles of the invention.

Fig. 1 illustrates a configuration of an X-ray image processing apparatus according to an embodiment.
Fig. 2 illustrates the detail of an image analysis processing unit that is the most featured configuration of the present invention.
Fig. 3 illustrates a processing flow by the image analysis processing unit.
Fig. 4 illustrates a flow of edge detection processing.
Fig. 5 illustrates a detailed configuration of a predetermined-region extracting unit.
Fig. 6 illustrates a flow of the predetermined-region extracting unit.
Fig. 7 illustrates an exemplary computer system that can provide the present invention.

## Description of Embodiments

Preferred embodiments of the present invention will be described in detail in accordance with the accompanying drawings.

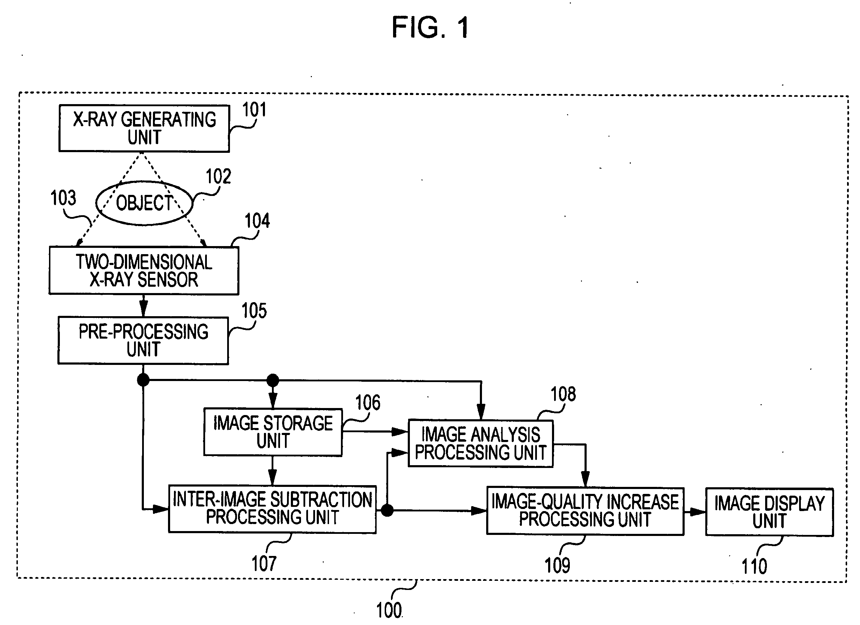

An X-ray image processing apparatus according to an embodiment of the present invention will be described below with reference to Fig. 1. An X-ray image processing apparatus 100 includes an X-ray generating unit 101 that can generate X-ray pulses by 3 to 30 pulses per second, and a two-dimensional X-ray sensor 104 that receives X-rays 103 transmitted through an object 102 and captures a movie that is synchronized with the X-ray pulses, as an X-ray image. The two-dimensional X-ray sensor 104 functions as an image pickup unit configured to capture a movie of the object 102 that is irradiated with the X-rays.

The X-ray image processing apparatus 100 includes a pre-processing unit 105 that performs pre-processing for respective frames of the movie captured by the two-dimensional X-ray sensor 104 at different times, and an image storage unit 106 that stores at least a single frame of the pre-processed movie as a mask image before a contrast agent is injected. The frame that is stored as the mask image is, for example, a frame immediately after the capturing of the movie is started, a frame immediately before the contrast agent is injected, the frame which is automatically acquired when the injection of the contrast agent is detected from the movie, or a frame that is selected because an operator instructs a storage timing when the injection of the contrast agent is started. Alternatively, a plurality of frames may be stored, and a frame used as the mask image may be properly selected or the plurality of frames may be combined. The X-ray image processing apparatus 100 includes an inter-image subtraction processing unit 107 that subtracts a mask image stored in the image storage unit 106 from a frame after the contrast agent is injected (hereinafter, referred to as "live image"), the X-ray image being output by the pre-processing unit 105, and that outputs the result as a subtraction image.

The X-ray image processing apparatus 100 includes an image analysis processing unit 108 that analyzes the subtraction image output by the inter-image subtraction processing unit 107 and at least one of the live image output by the pre-processing unit 105 and the mask image stored in the image storage unit 106, and extracts the boundary between the contrast-agent injection region and the background region.

The X-ray image processing apparatus 100 includes an image-quality increase processing unit 109 that performs image-quality increase processing for each frame on the basis of a boundary region between the contrast-agent injection region and the background region output by the image analysis processing unit 108, and an image display unit 110 that displays an image after the image-quality increase processing, as the DSA image. The image-quality increase processing performed here may be, for example, edge enhancement processing for boundary pixels extracted by the image analysis processing unit 108, noise reduction processing for pixels other than the boundary pixels, road mapping processing for superposing the boundary pixels on the live image, or gradation conversion processing.

In particular, a gradation conversion curve is generated by using a value, which is calculated from a pixel value in a region based on the contrast-agent injection region, as a parameter, and gradation conversion processing is performed for the subtraction image so that the contrast of the region based on the contrast-agent injection region is increased. Also, it is desirable to generate the gradation conversion curve by using a value, which is extracted from pixel values in the boundary region between the contrast-agent injection region and the background region, and to perform the gradation conversion processing for the subtraction region so that the contrast of the boundary region is increased. Accordingly, visibility is increased.

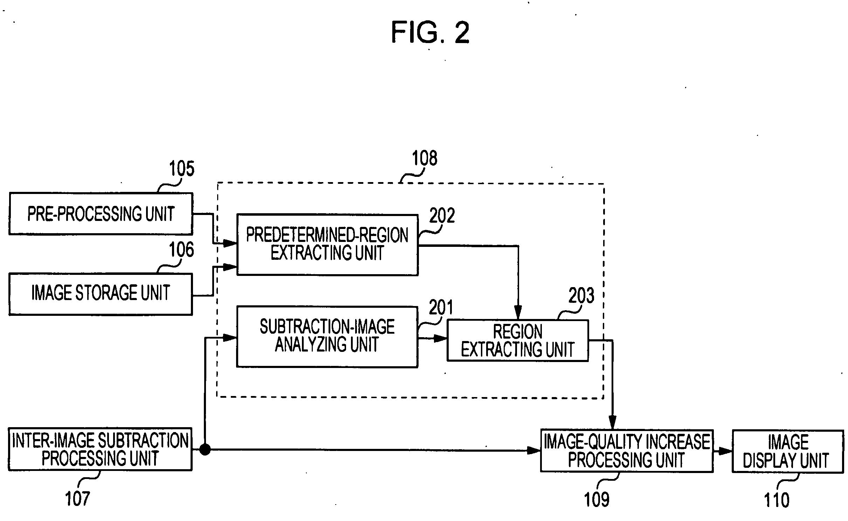

The configuration of the image analysis processing unit 108 is the most featured configuration in this embodiment, and will be described in detail with reference to a block diagram in Fig. 2.

The image analysis processing unit 108 includes a subtraction-image analyzing unit 201 that extracts a first region from the subtraction image, and a predetermined-region extracting unit 202 that extracts a second region from at least one of the mask image and the live image. The image analysis processing unit 108 also includes a region extracting unit 203 that extracts a region based on the contrast-agent injection region in accordance with the first region and the second region.

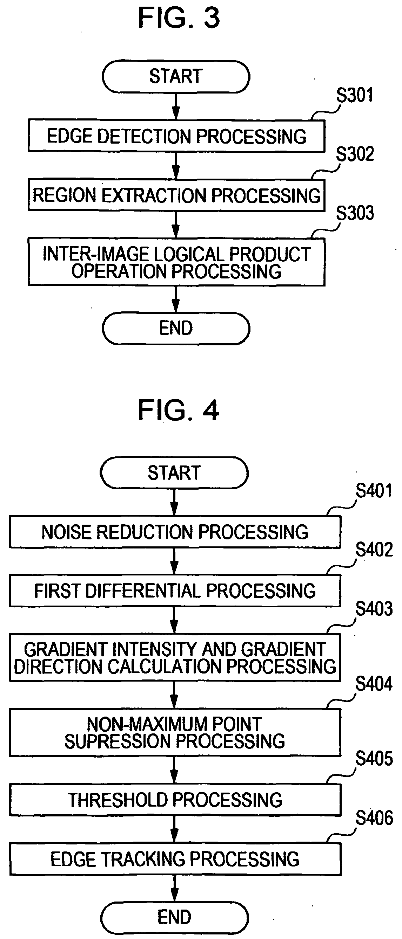

The operation of the image analysis processing unit 108 having the above configuration will be further described below with reference to a flowchart in Fig. 3.

In step S301, the image analysis processing unit 108 inputs the subtraction image output by the inter-image subtraction processing unit 107 to the subtraction-image analyzing unit 201. The subtraction-image analyzing unit 201 analyzes the subtraction image, and outputs a first binary image that represents the region based on the contrast-agent injection region. Here, the region based on the contrast-agent injection region is the contrast-agent injection region itself or a region included in the contrast-agent injection region and having a large change in contrast. Thus, the region based on the contrast-agent injection region includes a region that is obtained by information relating to the contrast-agent injection region. Also, the region based on the contrast-agent injection region includes a predetermined range of the contrast-agent injection region, the range which extends from the boundary between the contrast-agent injection region and the background region.

The first binary image is obtained on the basis of the contrast-agent injection region. In particular, extracting a region with a large change in contrast from the subtraction image is effective, because, if visibility of a region with a large change in contrast in the contrast-agent injection region is increased by, for example, gradation conversion or frequency processing, effectiveness of the diagnosis is increased. Also, in many cases, the region with the large change in contrast may frequently belong to a predetermined range in the contrast-agent injection region, the range which extends from the boundary between the contrast-agent injection region and a background region. Thus, this region is extracted.

The first binary image is an image including 1 indicative of an edge pixel that is obtained by performing edge detection processing for the subtraction image, and 0 indicative of the other pixel. In general, an edge obtained from the subtraction image by the edge detection processing includes an edge, which is not a subject for the extraction but is generated by a motion artifact appearing around a high or low pixel-value region, such as the boundary between the object and a transparent region, or around a lung field region or a metal region.

In step S302, the image analysis processing unit 108 inputs at least one of the live image output by the pre-processing unit 105 and the mask image stored in the image storage unit 106 to the predetermined-region extracting unit 202. The predetermined-region extracting unit 202 performs image analysis processing for the input image, and outputs a second binary image as a second region. The second binary image is an image including 1 indicative of a region to which the contrast agent may be injected, and 0 indicative of a high or low pixel-value region to which the contrast agent is not injected, such as the transparent region, the lung field region, or the metal region.

In step S303, the image analysis processing unit 108 inputs the first and second regions to the region extracting unit 203. The region extracting unit 203 generates a binary region image on the basis of the first and second regions. The binary region image is the output of the image analysis processing unit 108. The binary region image is obtained by performing inter-image logical product operation processing for the first and second binary images, and includes 1 indicative of a region extending from the region, to which the contrast agent is injected, to the region based on the contrast-agent injection region, and 0 indicative of the other region.

In this embodiment, various edge detection methods can be applied to the subtraction-image analyzing unit 201. The Canny edge detection method is an example of the edge detection method. Here, the operation of the subtraction-image analyzing unit 201 when the Canny edge detection method is used as the edge detection method will be described below with reference to a flowchart in Fig. 4.

In step S401, the subtraction-image analyzing unit 201 performs noise reduction processing for the subtraction image $I_S$ by a Gaussian filter, to generate a noise-reduced image $I_N$.

In step S402, the subtraction-image analyzing unit 201 performs first differential processing in the horizontal and vertical directions for the noise-reduced image IN to generate a horizontal differential image Gx and a vertical differential image Gy. In the first differential processing, an edge detecting operator, such as the Roberts operator, the Prewitt operator, or the Sobel operator, is used. The horizontal differential image Gx and the vertical differential image Gy are images whose pixel values have information about gradient intensities and gradient directions in the horizontal and vertical directions.

In step S403, the subtraction-image analyzing unit 201 calculates a gradient intensity image G and a gradient direction image θ with the following expressions by using the horizontal differential image Gx and the vertical differential image Gy.

[Math. 1]

$$G = \sqrt{Gx^2 + Gy^2}$$

[Math. 2]

$$\theta = \arctan\left(\frac{Gy}{Gx}\right)$$

The gradient intensity image G is an image in which pixel values represent gradient intensities. The gradient direction image θ is an image in which pixel values represent gradient directions such that, for example, in the noise-reduced image IN, the gradient directions are expressed by values in a range from $-n/2$ (inclusive) to $\pi/2$ (exclusive), the values including 0 indicative of a pixel whose pixel value increases in the horizontal direction and n/2 indicative of a pixel whose pixel value increases in the vertical direction.

In step S404, the subtraction-image analyzing unit 201 performs non-maximum point suppression processing based on the gradient intensity image G and the gradient direction image θ, and outputs a potential edge image E as edge information. The potential edge image E is a binary image including 1 indicative of a local maximum edge pixel in the noise-reduced image and 0 indicative of the other pixel. In the non-maximum point suppression processing, two adjacent pixels of a target pixel (x, y) are selected on the basis of the gradient direction image θ(x, y). If a gradient intensity image G(x, y) of the target pixel (x, y) is larger than the values of the two adjacent pixels, the target pixel (x, y) is considered as a local maximum edge pixel, and is expressed as E(x, y) = 1. A specific example is described as follows.

If the gradient direction image θ(x, y) is in a range from $-\pi/8$ (inclusive) to $\pi/8$ (exclusive), E(x, y) is determined by the following expression while two pixels arranged in the horizontal direction serve as the adjacent pixels.

[Math. 3]

$$E(x, y) = \begin{cases} 1 & (G(x-1, y) < G(x, y) \, and \, G(x, y) > G(x+1, y)) \\ 0 & \text{(Other than those above)} \end{cases}$$

If the gradient direction image θ(x, y) is in a range from $\pi/8$ (inclusive) to $3\pi/8$ (exclusive), E(x, y) is determined by the following expression while two pixels arranged in an oblique direction serve as the adjacent pixels.

[Math. 4]

$$E(x,y) = \begin{cases} 1 & (G(x,y) > G(x-1,y-1) \, and \, G(x,y) > G(x+1,y+1)) \\ 0 & \text{(Other than those above)} \end{cases}$$

If the gradient direction image $\theta(x, y)$ is in a range from $3\pi/8$ (inclusive) to $\pi/2$ (exclusive) or a range from $-n/2$ (inclusive) to $-3\pi/8$ (exclusive), E(x, y) is determined by the following expression while two pixels arranged in the vertical direction serve as the adjacent pixels.

[Math. 5]

$$E(x,y) = \begin{cases} 1 & (G(x,y) > G(x,y-1) \, and \, G(x,y) > G(x,y+1)) \\ 0 & \text{(Other than those above)} \end{cases}$$

If the gradient direction image $\theta(x, y)$ is in a range from $-3\pi/8$ (inclusive) to $-\pi/8$ (exclusive), E(x, y) is determined by the following expression while two pixels arranged in an oblique direction serve as the adjacent pixels.

[Math. 6]

$$E(x,y) = \begin{cases} 1 & (G(x,y) > G(x-1,y+1) \, and \, G(x,y) > G(x+1,y-1)) \\ 0 & \text{(Other than those above)} \end{cases}$$

In step S405, the subtraction-image analyzing unit 201 performs threshold processing for the potential edge image E on the basis of the gradient intensity image G and two thresholds $T_{low}$ and $T_{high}$ ($T_{low} < T_{high}$), and outputs a low edge image $E_{low}$ and a high edge image $E_{high}$. The low edge image $E_{low}$ is a binary image including, when gradient intensity images G(x, y) are respectively compared with values $T_{low}$ for all pixels (x, y) that satisfy potential edge image E(x, y) = 1, 1 indicative of a pixel that satisfies G(x, y) > $T_{low}$ and 0 indicative of the other pixel. The high edge image $E_{high}$ is a binary image including, when gradient intensity images G(x, y) are respectively compared with values $T_{high}$ for all pixels (x, y) that satisfy potential edge image E(x, y) = 1, 1 indicative of a pixel that satisfies G(x, y) > $T_{high}$, and 0 indicative of the other pixel.

In step S406, the subtraction-image analyzing unit 201 performs edge tracking processing on the basis of the low edge image $E_{low}$ and the high edge image $E_{high}$, and outputs an edge image $I_E$. In the edge tracking processing, if a connected component of the pixels (x, y) that satisfy low edge image $E_{low}$(x, y) = 1 includes pixels (x, y) that satisfy high edge image $E_{high}$ (x, y) = 1, all pixels (x, y) included in the connected component are considered as edge pixels, which are expressed by $I_E$(x, y) = 1. The other pixels (x, y) are non-edge pixels, which are expressed by $I_E$(x, y) = 0. The edge image $I_E$ acquired by the above processing is output as the result of the Canny edge detection method, and the Canny edge detection processing is ended.

The boundary between the contrast-agent injection region and the background region, the boundary which is a subject for the edge detection according to this embodiment, has an edge characteristic that varies depending on the injection state of the contrast agent. Hence, in the edge detection processing, the operator used for the noise reduction processing or the first differential processing may be properly changed depending on the time since the injection of the contrast agent is started. If the frame rate during the image capturing is high, to increase the processing speed, part of the noise reduction processing, threshold processing, or edge tracking processing may be omitted or replaced with relatively simple processing. Another example of the edge detection processing may be the zero-crossing method for detecting zero-crossing on the basis of second differential processing.

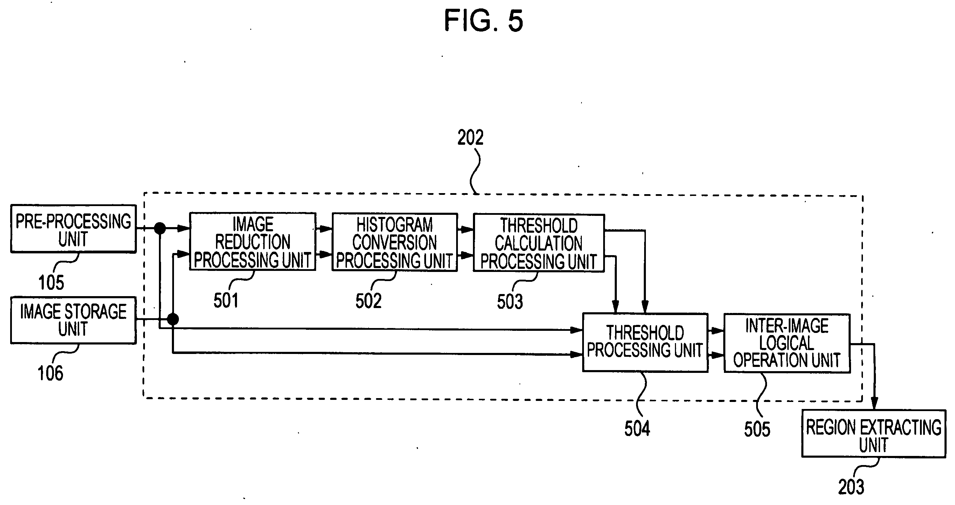

In this embodiment, various analyzing methods can be applied to the predetermined-region extracting unit 202 for the mask image and the live image which are subjects for the analysis. Fig. 5 is a block diagram showing the predetermined-region extracting unit 202 including an image reducing unit 501, a histogram conversion processing unit 502, a threshold calculation processing unit 503, a threshold processing unit 504, and an inter-image logical operation unit 505.

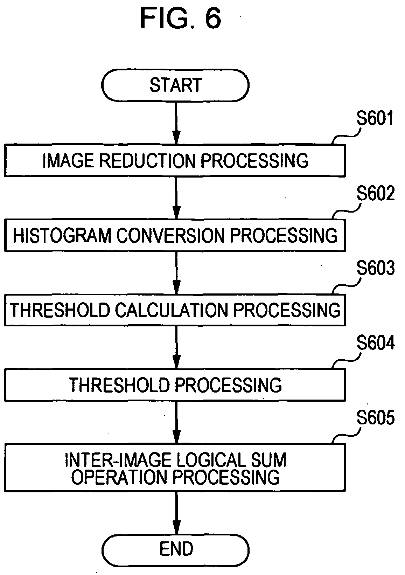

Here, with this configuration, a method for generating the binary region image including 0 indicative of the high pixel-value region such as the lung field and the transparent region and 1 indicative of the other region will be described with reference to a flowchart in Fig. 6.

In step S601, the predetermined-region extracting unit 202 inputs the live image $I_L$ output by the pre-processing unit 105 and the mask image $I_M$ stored in the image storage unit 106 to the image reducing unit 501. The image reducing unit 501 performs image reduction processing for these images, and outputs a reduced live image $I_L'$ and a reduced mask image $I_M'$. For example, the image reduction processing is performed such that an image is divided into blocks each having a plurality of pixels, and an average value of each block is determined as a pixel value of a single pixel of a reduced image.

In step S602, the predetermined-region extracting unit 202 inputs the reduced live image $I_L'$ and the reduced mask image $I_M'$ to the histogram conversion processing unit 502. The histogram conversion processing unit 502 generates and outputs a live-image pixel-value histogram $H_L$ and a mask-image pixel-value histogram $H_M$. Each pixel-value histogram is generated by counting the number of pixels in an image for every predetermined pixel-value range.

In step S603, the predetermined-region extracting unit 202 inputs the live-image pixel-value histogram $H_L$ and the mask-image pixel-value histogram $H_M$ to the threshold calculation processing unit 503. The threshold calculation processing unit 503 outputs a live-image binarization threshold $T_L$ and a mask-image binarization threshold $T_M$. Each threshold serving as a predetermined value is calculated by using a phenomenon that, in the live or mask image, the lung field or the transparent region, which is a high pixel-value region, generates a peak of a histogram. For example, a histogram for the high pixel-value region may be scanned from a pixel value with a maximum frequency in the histogram in a low pixel-value direction, and a pixel value with a minimum frequency that is found first may be acquired as the threshold serving as the predetermined value.

In step S604, the predetermined-region extracting unit 202 inputs the live image $I_L$ and the mask image $I_M$ to the threshold processing unit 504. The threshold processing unit 504 performs threshold processing based on the live-image binarization threshold $T_L$ and the mask-image binarization threshold $T_M$, and outputs a binary live image $B_L$ and a binary mask image $B_M$. The binary live image $B_L$ and the binary mask image $B_M$ are obtained by comparing all pixels in the live image $I_L$ and the mask image $I_M$ with the corresponding binarization thresholds $T_L$ and $T_M$, so that images include 0 indicative of pixel values equal to or larger than the thresholds and 1 indicative of the other pixel.

Since the region, to which the contrast agent is not injected, is eliminated from each of the live image $I_L$ and the mask image $I_M$, the motion artifact resulted from the motion of the object can be suppressed. Hence, an unnecessary edge is not detected. Also, a region with a large difference between pixel values, such as the boundary between the object and the transparent region, or the boundary between the lung field region or the metal region and the other region, is not erroneously detected.

In step S605, the predetermined-region extracting unit 202 inputs the binary live image $B_L$ and the binary mask image $B_M$ to the inter-image logical operation unit 505. The inter-image logical operation unit 505 performs inter-image logical sum operation and outputs the calculation result as a binary region image B. The binary region image B is an image having a pixel value based on the logical sum of corresponding pixels in the binary live image $B_L$ and the binary mask image $B_M$. The binary region image B serves as the output of the predetermined-region extracting unit 202. The flow is ended.

In the above embodiment, the processing for extracting the high pixel-value region, such as the lung field or the transparent region, is described. Processing similar thereto may be applied to extraction for a low pixel-value region, such as the metal region or a region outside an irradiation field, which does not include the boundary between the contrast-agent injection region and the background region.

Alternatively, a region, which is not a subject for the extraction, may be extracted by clustering through pattern recognition processing with a discriminator, such as the neural network, support vector machine, or Bayesian classifier. In this case, for example, learning for the region not including the boundary between the contrast-agent injection region and the background region may be performed for each object portion and posture of the image capturing. Thus, the region having complex characteristics can be defined as the region which is not a subject for the extraction, rather than the method based on the threshold processing or the histogram analysis.

The first and second regions output by the subtraction-image analyzing unit 201 and the predetermined-region extracting unit 202 are binary images. Thus, the regions can be combined by the inter-image logical product operation. The region extracting unit 203 performs the inter-image logical product operation for the two binary images,.and hence generates a binary boundary image including 1 indicative of an edge which is a subject for the extraction and 0 indicative of the other edge.

The binary boundary image is input to the image-quality increase processing unit 109, which is located at the downstream side, as the output of the image analysis processing unit 108. The image-quality increase processing unit 109 performs image processing for the subtraction image or the live image with reference to the binary boundary image. For example, the image-quality increase processing unit 109 applies sharpening processing and contrast enhancement processing to pixels whose pixel values in the binary boundary image are 1, and applies noise reduction processing to pixels whose pixel values in the binary boundary image are 0. Also, if the pixel values of the pixels in the subtraction image corresponding to the pixel values being 1 of the pixels in the binary boundary image are added to the live image, a road map image, on which the boundary of the contrast agent in the live image is superposed, can be generated.

With this embodiment, the extraction processing for the boundary between the contrast agent and the background, the boundary which is used for the image-quality increase processing for the DSA image, is performed such that the edge including a noise is detected from the subtraction image, and the region, to which the contrast agent is not injected, is extracted from each of the mask image and the live image before the subtraction. The region, to which the contrast agent is not injected, is extracted on the basis of the pixel values of the image eliminated from the subtraction image. The region can be used for reducing the noise region from the edge. By using the region, to which the contrast agent is not injected, for the extraction processing for the boundary between the contrast agent and the background, the processing accuracy can be increased.

In this embodiment, the binary region information is extracted through the analysis of both the mask image and the live image. However, the binary region information may be obtained in advance through analysis of only the mask image as a subject for the analysis. In this case, only required is analysis for the subtraction image during the movie capturing. Thus, high-speed processing can be performed.

The units shown in Figs. 1 and 2 may be formed by dedicated hardware configurations. Alternatively, functions of the hardware configurations may be provided by software. In this case, the functions of the units shown in Figs. 1 and 2 can be provided by installing the software in an information processing device and by executing the software to provide an image processing method through an arithmetic operation function of the information processing device. Through the execution of the software, for example, the mask image and the live image before and after the contrast agent is injected are acquired by the pre-processing for respective frames of the movie output by the two-dimensional X-ray sensor 104, and the subtraction image is acquired by the inter-image subtracting step. Then, the image analyzing step including the first region extraction from the subtraction image, the second region extraction from the mask image and the live image, and the extracting step for the boundary between the contrast agent and the background; and the image-quality increasing step using the image analysis result are executed.

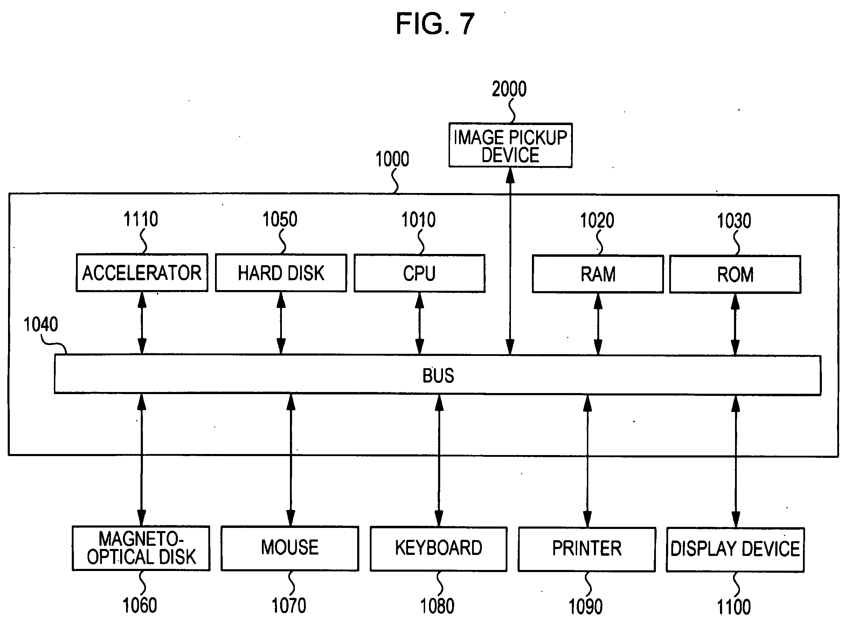

Fig. 7 is a block diagram showing a hardware configuration of the information processing device and peripheral devices thereof. An information processing device 1000 is connected with an image pickup device 2000 such that data communication can be made therebetween.

With this embodiment, the extraction processing for the boundary between the contrast agent and the background used for the image-quality increase processing for the DSA image is performed on the basis of the image analysis processing for the subtraction image and the image analysis processing for at least one of the mask image and the live image before the subtraction. The information eliminated from the subtraction image is acquired from at least one of the mask image and the live image, and is used for the extraction processing for the boundary between the contrast agent and the background. Accordingly, the processing accuracy can be increased.

Also, in general, since the mask image is not changed by single DSA inspection, the result of single image analysis can be reused. Further, although the image analysis processing applied for each of the mask image, the live image, and the subtraction image is relatively simple processing, the processing can finally provide a large amount of information. Thus, high-speed processing can be performed without complex processing.

Further, with the X-ray image processing apparatus, to which the image-quality increase processing with the use of the extraction processing for the boundary between the contrast agent and the background is applied, the increase in quality of the DSA image and the increase in speed for acquiring the DSA image can be provided. Thus, diagnostic performance for angiographic inspection can be increased.

**Information Processing Device**

A CPU 1010 controls the entire information processing device 1000 by using a program and data stored in a RAM 1020 and a ROM 1030. Also, the CPU 1010 executes arithmetic processing relating to image processing that is predetermined by the execution of the program.

The RAM 1020 includes an area for temporarily storing a program and data loaded from a magneto-optical disk 1060 or a hard disk 1050. The RAM 1020 also includes an area for temporarily storing image data such as the mask image, live image, and subtraction image, acquired from the image pickup device 2000. The RAM 1020 further includes a work area that is used when the CPU 1010 executes various processing. The ROM 1030 stores setting data and a boot program of the information processing device 1000.

The hard disk 1050 holds an operating system (OS), and a program and data for causing the CPU 1010 included in the computer, to execute the processing of the respective units shown in Figs. 1 and 2. The held contents are loaded to the RAM 1020 properly under the control by the CPU 1010, and become subjects for the processing by the CPU 1010 (computer). In addition, the hard disk 1050 can save the data of the mask image, live image, and subtraction image.

The magneto-optical disk 1060 is an example of an information storage medium. The magneto-optical disk 1060 can store part of or all the program and data saved in the hard disk 1050.

When an operator of the information processing device 1000 operates a mouse 1070 or a keyboard 1080, the mouse 1070 or the keyboard 1080 can input various instructions to the CPU 1010.

A printer 1090 can print out an image, which is displayed on the image display unit 110, onto a recording medium.

A display device 1100 is formed of a CRT or a liquid crystal screen. The display device 1100 can display the processing result of the CPU 1010 by images and characters. For example, the display device 1100 can display the image processed by the respective units shown in Figs. 1 and 2 and finally output from the image display unit 110. In this case, the image display unit 110 functions as a display control unit configured to cause the display device 1100 to display an image. A bus 1040 connects the respective units in the information processing device 1000 with each other to allow the respective units to exchange data.

**Image Pickup Device**

Next, the image pickup device 2000 will be described. The image pickup device 2000 can capture the movie during the injection of the contrast agent like the X-ray fluoroscopy apparatus. The image pickup device 2000 transmits the captured image data to the information processing device 1000. Plural pieces of image data may be collectively transmitted to the information processing device 1000. Alternatively, image data may be transmitted successively in order of capturing.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1.   An image processing apparatus (100) comprising:

   a subtracting unit (107) configured to acquire a subtraction image by performing subtraction processing among a plurality of radiographic images that are obtained when an image of an object is captured at different times;
   a subtraction-image analyzing unit (201) configured to extract a first region from the subtraction image;
   a predetermined-region extracting unit (202) configured to extract a second region from one of the plurality of radiographic images; and
   a region extracting unit (108, 203) configured to extract, based on the first region extracted by the subtraction-image analyzing unit (201) and the second region extracted by the predetermined-region extracting unit (202), a region based on a contrast-agent injection region.

2.   The image processing apparatus according to claim 1, wherein the predetermined-region extracting unit (202) is operable to acquire the contrast-agent injection region from each of an image before a contrast agent is injected and an image after the contrast image is injected, and extracts a region, in which the acquired regions are overlapped with each other, as the second region.

3.   The image processing apparatus according to claim 1, wherein the predetermined-region extracting unit (202) is operable to extract the contrast-agent injection region from one of an image before a contrast agent is injected and an image after the contrast agent is injected, as the second region.

4.   The image processing apparatus according to any one of claims 1 to 3,
   wherein the subtraction-image analyzing unit (201) is operable to perform edge detection processing for the subtraction image, and is operable to acquire the first region based on the contrast-agent injection region from edge information obtained as the result of the edge detection processing.

5.   The image processing apparatus according to claim 4, wherein the subtraction-image analyzing unit (201) is operable to use the Canny edge detection method as the edge detection processing for the subtraction image.

6.   The image processing apparatus according to claim 4, wherein the subtraction-image analyzing unit (201) is operable to use the zero-crossing method as the edge detection processing for the subtraction image.

7.   The image processing apparatus according to any one of claims 1 to 6, wherein the subtraction-image analyzing unit (201) is operable to include a plurality of edge detecting operators for the edge detection processing for the subtraction image, and is operable to select one of the edge detecting operators in accordance with an injection state of the contrast agent.

8. The image processing apparatus according to any one of claims 1 to 7, wherein the predetermined-region extracting unit (202) is operable to extract a high pixel-value region or a low pixel-value region from at least one of the images before the contrast agent is injected and the images after the contrast agent is injected on the basis of comparison with a predetermined value.

9. The image processing apparatus according to any one of claims 1 to 7,
wherein the predetermined-region extracting unit (202) includes
a histogram converting unit (501) configured to generate a pixel-value histogram from at least one of the images before the contrast agent is injected and the images after the contrast agent is injected, and
a threshold calculating unit (503) configured to analyzes the pixel-value histogram and calculates a threshold, and
wherein the predetermined-region extracting unit (202) extracts the second region on the basis of the threshold.

10. The image processing apparatus according to any one of claims 1 to 9, further comprising an image-quality increase processing unit (109) configured to generate a gradation conversion curve by using a value, which is calculated from a pixel value of the region based on the contrast-agent injection region extracted by the region extracting unit, as a parameter, and to perform gradation conversion processing for the subtraction image such that a contrast of the region based on the contrast agent injection region is increased.

11. An image processing method comprising:

a subtracting step of acquiring a subtraction image by performing subtraction processing among a plurality of radiographic images that are obtained when an image of an object is captured at different times;
a subtraction-image analyzing step of extracting a first region from the subtraction image;
a predetermined region extracting step of extracting a second region from one of the plurality of radiographic images; and
a region extracting step of extracting, based on the first region and the second region, a region based on a contrast-agent injection region.

12. A storage medium (1050, 1060) storing a program that causes a computer (1000) to execute the image processing method according to claim 11.

13. The image processing apparatus according to any one of claims 1-10, wherein:

the subtraction-image analyzing unit (201) is configured to extract the first region based on a contrast-agent injection region from the subtraction image; and
the predetermined-region extracting unit (202) is configured to extract the second region based on a contrast-agent injection region from at least any one of the plurality of radiographic images.

## Patentansprüche

1. Bildverarbeitungsvorrichtung (100) umfassend:

eine Subtraktionseinheit (107), die konfiguriert ist, ein Subtraktionsbild durch Durchführen von Subtraktionsverarbeitung unter mehreren radiografischen Bildern zu erfassen, die erhalten werden, wenn zu verschiedenen Zeitpunkten ein Bild eines Objekts aufgenommen wird;
eine Subtraktionsbildanalyseeinheit (201), die konfiguriert ist, eine erste Region aus dem Subtraktionsbild zu extrahieren;
eine Extraktionseinheit für eine vorbestimmte Region (202), die konfiguriert ist, eine zweite Region aus einem der mehreren radiografischen Bilder zu extrahieren; sowie
eine Regionsextraktionseinheit (108, 203), die konfiguriert ist, basierend auf der durch die Subtraktionsbildanalyseeinheit (201) extrahierten ersten Region und der durch die Extraktionseinheit für eine vorbestimmte Region (202) extrahierten zweiten Region eine auf einer Kontrastmittelinjektionsregion basierende Region zu extrahieren.

**2.** Bildverarbeitungsvorrichtung nach Anspruch 1,

wobei die Extraktionseinheit für eine vorbestimmte Region (202) betreibbar ist, die Kontrastmittelinjektionsregion sowohl aus einem Bild vor einer Kontrastmittelinjektion als auch aus einem Bild nach der Kontrastmittelinjektion zu erfassen sowie eine Region, in der die erfassten Regionen einander überlappen, als die zweite Region zu extrahieren.

**3.** Bildverarbeitungsvorrichtung nach Anspruch 1,

wobei die Extraktionseinheit für eine vorbestimmte Region (202) betreibbar ist, die Kontrastmittelinjektionsregion aus einem von einem Bild vor einer Kontrastmittelinjektion und einem Bild nach der Kontrastmittelinjektion als die zweite Region zu extrahieren.

**4.** Bildverarbeitungsvorrichtung nach einem der Ansprüche 1 bis 3,

wobei die Subtraktionsbildanalyseeinheit (201) betreibbar ist, Kantendetektionsverarbeitung am Subtraktionsbild durchzuführen, und weiterhin betreibbar ist, die erste Region basierend auf der Kontrastmittelinjektionsregion aus als das Ergebnis der Kantendetektionsverarbeitung erhaltener Kanteninformation zu erfassen.

**5.** Bildverarbeitungsvorrichtung nach Anspruch 4,

wobei die Subtraktionsbildanalyseeinheit (201) betreibbar ist, das Canny-Kantendetektionsverfahren als die Kantendetektionsverarbeitung am Subtraktionsbild zu verwenden.

**6.** Bildverarbeitungsvorrichtung nach Anspruch 4,

wobei die Subtraktionsbildanalyseeinheit (201) betreibbar ist, das Nulldurchgangsverfahren als die Kantendetektionsverarbeitung am Subtraktionsbild zu verwenden.

**7.** Bildverarbeitungsvorrichtung nach einem der Ansprüche 1 bis 6,

wobei die Subtraktionsbildanalyseeinheit (201) betreibbar ist, mehrere Kantendetektionsoperatoren für die Kantendetektionsverarbeitung am Subtraktionsbild zu beinhalten, und weiterhin betreibbar ist, einen der Kantendetektionsoperatoren gemäß einem Kontrastmittelinjektionszustand auszuwählen.

**8.** Bildverarbeitungsvorrichtung nach einem der Ansprüche 1 bis 7,

wobei die Extraktionseinheit für eine vorbestimmte Region (202) betreibbar ist, eine Hochpixelwertregion oder eine Niedrigpixelwertregion aus zumindest einem der Bilder vor der Kontrastmittelinjektion und der Bilder nach der Kontrastmittelinjektion auf der Basis eines Vergleichs mit einem vorbestimmten Wert zu extrahieren.

**9.** Bildverarbeitungsvorrichtung nach einem der Ansprüche 1 bis 7,

wobei die Extraktionseinheit für eine vorbestimmte Region (202) beinhaltet
eine Histogrammumwandlungseinheit (501), die konfiguriert ist, ein Pixelwerthistogramm aus zumindest einem der Bilder vor der Kontrastmittelinjektion und der Bilder nach der Kontrastmittelinjektion zu erzeugen, sowie eine Schwellenwertberechnungseinheit (503), die konfiguriert ist, das Pixelwerthistogramm zu analysieren und einen Schwellenwert zu berechnen; und
wobei die Extraktionseinheit für eine vorbestimmte Region (202) die zweite Region auf der Basis des Schwellenwerts extrahiert.

**10.** Bildverarbeitungsvorrichtung nach einem der Ansprüche 1 bis 9,

weiterhin umfassend eine Verarbeitungseinheit für Bildqualitätsvergrößerung (109), die konfiguriert ist, eine Gradationsumwandlungskurve zu erzeugen durch Verwenden eines Werts als Parameter, wobei der Wert aus einem Pixelwert der auf der Basis der Kontrastmittelinjektionsregion durch die Regionsextraktionseinheit extrahierten Region berechnet wird, sowie Gradationsumwandlungsverarbeitung am Subtraktionsbild so durchzuführen, dass ein Kontrast der auf der Kontrastmittelinjektionsregion basierenden Region vergrößert wird.

**11.** Bildverarbeitungsverfahren umfassend:

einen Subtraktionsschritt zum Erfassen eines Subtraktionsbilds durch Durchführen von Subtraktionsverarbeitung unter mehreren radiografischen Bildern, die erhalten werden, wenn zu verschiedenen Zeitpunkten ein Bild eines Objekts aufgenommen wird;
einen Subtraktionsbildanalyseschritt zum Extrahieren einer ersten Region aus dem Subtraktionsbild;
einen Extraktionsschritt für eine vorbestimmte Region zum Extrahieren einer zweiten Region aus einem der mehreren radiografischen Bilder; sowie
einen Regionsextraktionsschritt zum basierend auf der ersten Region und der zweiten Region Extrahieren einer auf einer Kontrastmittelinjektionsregion basierenden Region.

**12.** Speichermedium (1050, 1060), das ein Programm speichert, das einen Computer (1000) veranlasst, das Bildverarbeitungsverfahren nach Anspruch 11 auszuführen.

**13.** Bildverarbeitungsvorrichtung nach einem der Ansprüche 1-10, wobei:

die Subtraktionsbildanalyseeinheit (201) konfiguriert ist, die erste Region basierend auf einer Kontrastmittelinjektionsregion aus dem Subtraktionsbild zu extrahieren; und
die Extraktionseinheit für eine vorbestimmte Region (202) konfiguriert ist, die zweite Region basierend auf einer Kontrastmittelinjektionsregion aus zumindest irgendeinem der mehreren radiografischen Bilder zu extrahieren.

**Revendications**

**1.** Appareil de traitement d'image (100) comprenant :

une unité de soustraction (107) configurée pour acquérir une image de soustraction en effectuant un traitement de soustraction parmi une pluralité d'images radiographiques qui sont obtenues lorsqu'une image d'un objet est capturée à différents instants ;
une unité d'analyse d'image de soustraction (201) configurée pour extraire une première région de l'image de soustraction ;
une unité d'extraction de région prédéterminée (202) configurée pour extraire une seconde région de l'une de la pluralité d'images radiographiques ; et
une unité d'extraction de région (108, 203) configurée pour extraire, sur la base de la première région extraite par l'unité d'analyse d'image de soustraction (201) et de la seconde région extraite par l'unité d'extraction de région prédéterminée (202), une région basée sur une région d'injection d'agent de contraste.

**2.** Appareil de traitement d'image selon la revendication 1, dans lequel l'unité d'extraction de région prédéterminée (202) a pour fonction d'acquérir la région d'injection d'agent de contraste à partir de chacune d'une image obtenue avant injection d'un agent de contraste et d'une image obtenue après injection de l'agent de contraste, et extrait une région dans laquelle les régions acquises se superposent les unes aux autres, en tant que seconde région.

**3.** Appareil de traitement d'image selon la revendication 1, dans lequel l'unité d'extraction de région prédéterminée (202) a pour fonction d'extraire la région d'injection d'agent de contraste de l'une d'une image obtenue avant injection d'un agent de contraste et d'une image obtenue après injection de l'agent de contraste, en tant que seconde région.

**4.** Appareil de traitement d'image selon l'une quelconque des revendications 1 à 3,
dans lequel l'unité d'analyse d'image de soustraction (201) a pour fonction d'effectuer un traitement de détection de bord sur l'image de soustraction et a pour fonction d'acquérir la première région sur la base de la région d'injection d'agent de contraste à partir d'informations de bord obtenues comme résultat du traitement de détection de bord.

**5.** Appareil de traitement d'image selon la revendication 4, dans lequel l'unité d'analyse d'image de soustraction (201) a pour fonction d'utiliser un procédé de détection de bord de Canny en tant que traitement de détection de bord effectué sur l'image de soustraction.

**6.** Appareil de traitement d'image selon la revendication 4, dans lequel l'unité d'analyse d'image de soustraction (201) a pour fonction d'utiliser un procédé de passage à zéro en tant que traitement de détection de bord effectué sur l'image de soustraction.

7. Appareil de traitement d'image selon l'une quelconque des revendications 1 à 6, dans lequel l'unité d'analyse d'image de soustraction (201) a pour fonction d'inclure une pluralité d'opérateurs de détection de bord pour le traitement de détection de bord effectué sur l'image de soustraction et a pour fonction de sélectionner l'un des opérateurs de détection de bord conformément à un état d'injection de l'agent de contraste.

8. Appareil de traitement d'image selon l'une quelconque des revendications 1 à 7, dans lequel l'unité d'extraction de région prédéterminée (202) a pour fonction d'extraire une région à valeurs de pixels élevées ou une région à faible valeur de pixels d'au moins l'une des images obtenues avant injection de l'agent de contraste et des images obtenues après injection de l'agent de contraste sur la base d'une comparaison à une valeur prédéterminée.

9. Appareil de traitement d'image selon l'une quelconque des revendications 1 à 7,
dans lequel l'unité d'extraction de région prédéterminée (202) comporte
une unité de conversion d'histogramme (501) configurée pour générer un histogramme de valeurs de pixels à partir d'au moins l'une des images obtenues avant injection de l'agent de contraste et des images obtenues après injection de l'agent de contraste, et
une unité de calcul de seuil (503) configurée pour analyser l'histogramme de valeurs de pixels et calculer un seuil, et
dans lequel l'unité d'extraction de région prédéterminée (202) extrait la seconde région sur la base du seuil.

10. Appareil de traitement d'image selon l'une quelconque des revendications 1 à 9, comprenant en outre une unité de traitement d'augmentation de la qualité d'image (109) configurée pour générer une courbe de conversion de gradation en utilisant une valeur qui est calculée à partir d'une valeur de pixel de la région basée sur la région d'injection d'agent de contraste extraite par l'unité d'extraction de région, en tant que paramètre, et pour effectuer un traitement de conversion de gradation sur l'image de soustraction de façon à augmenter un contraste de la région basée sur la région d'injection d'agent de contraste.

11. Procédé de traitement d'image comprenant :

une étape de soustraction consistant à acquérir une image de soustraction en effectuant un traitement de soustraction parmi une pluralité d'images radiographiques qui sont obtenues lorsqu'une image d'un objet est capturée à différents instants ;
une étape d'analyse d'image de soustraction consistant à extraire une première région de l'image de soustraction ;
une étape d'extraction de région prédéterminée consistant à extraire une seconde région de l'une de la pluralité d'images radiographiques ; et
une étape d'extraction de région consistant à extraire, sur la base de la première région et de la seconde région, une région basée sur une région d'injection d'agent de contraste.

12. Support de stockage (1050, 1060) stockant un programme qui amène un ordinateur (1000) à exécuter le procédé de traitement d'image selon la revendication 11.

13. Appareil de traitement d'image selon l'une quelconque des revendications 1-10, dans lequel :

l'unité d'analyse d'image de soustraction (201) est configurée pour extraire la première région basée sur une région d'injection d'agent de contraste de l'image de soustraction ; et
l'unité d'extraction de région prédéterminée est configurée pour extraire la seconde région basée sur la région d'injection d'agent de contraste d'au moins l'une quelconque de la pluralité d'images radiographiques.

# FIG. 1

# FIG. 2

# FIG. 3

```
        ┌──────────┐
        │  START   │
        └────┬─────┘
             │                        S301
        ┌────▼─────────────────────┐
        │ EDGE DETECTION PROCESSING │
        └────┬─────────────────────┘
             │                        S302
        ┌────▼─────────────────────────┐
        │ REGION EXTRACTION PROCESSING  │
        └────┬─────────────────────────┘
             │                        S303
        ┌────▼──────────────────────────┐
        │ INTER-IMAGE LOGICAL PRODUCT    │
        │    OPERATION PROCESSING        │
        └────┬──────────────────────────┘
             │
        ┌────▼─────┐
        │   END    │
        └──────────┘
```

# FIG. 4

```
        ┌──────────┐
        │  START   │
        └────┬─────┘
             │                        S401
        ┌────▼─────────────────────┐
        │ NOISE REDUCTION PROCESSING │
        └────┬─────────────────────┘
             │                        S402
        ┌────▼─────────────────────────┐
        │ FIRST DIFFERENTIAL PROCESSING │
        └────┬─────────────────────────┘
             │                        S403
        ┌────▼────────────────────────────┐
        │ GRADIENT INTENSITY AND GRADIENT  │
        │ DIRECTION CALCULATION PROCESSING │
        └────┬────────────────────────────┘
             │                        S404
        ┌────▼─────────────────────┐
        │    NON-MAXIMUM POINT       │
        │  SUPRESSION PROCESSING     │
        └────┬─────────────────────┘
             │                        S405
        ┌────▼─────────────────────┐
        │   THRESHOLD PROCESSING    │
        └────┬─────────────────────┘
             │                        S406
        ┌────▼─────────────────────┐
        │  EDGE TRACKING PROCESSING  │
        └────┬─────────────────────┘
             │
        ┌────▼─────┐
        │   END    │
        └──────────┘
```

# FIG. 5

# FIG. 6

```
          ┌─────────────┐
          │    START    │
          └──────┬──────┘
                 │              S601
                 ▼
   ┌──────────────────────────────┐
   │  IMAGE REDUCTION PROCESSING   │
   └───────────────┬──────────────┘
                   │            S602
                   ▼
   ┌──────────────────────────────────┐
   │ HISTOGRAM CONVERSION PROCESSING  │
   └───────────────┬──────────────────┘
                   │            S603
                   ▼
   ┌──────────────────────────────────┐
   │ THRESHOLD CALCULATION PROCESSING │
   └───────────────┬──────────────────┘
                   │            S604
                   ▼
   ┌──────────────────────────────┐
   │     THRESHOLD PROCESSING     │
   └───────────────┬──────────────┘
                   │            S605
                   ▼
   ┌──────────────────────────────┐
   │   INTER-IMAGE LOGICAL SUM    │
   │    OPERATION PROCESSING      │
   └───────────────┬──────────────┘
                   │
                   ▼
          ┌─────────────┐
          │     END     │
          └─────────────┘
```

# FIG. 7

ACCELERATOR 1110 — HARD DISK 1050 — CPU 1010 — RAM 1020 — ROM 1030 — BUS 1040 — IMAGE PICKUP DEVICE 2000 — MAGNETO-OPTICAL DISK 1060 — MOUSE 1070 — KEYBOARD 1080 — PRINTER 1090 — DISPLAY DEVICE 1100 — 1000

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4030786 A

- JP 5167927 A

**Non-patent literature cited in the description**

- **PHILIPPE GAILLOUD et al.** Three-Dimensional Fusion Digital Subtraction Angiography: New Reconstruction Algorithm for Simultaneous Three-Dimensional Rendering of Osseous and Vascular Information Obtained during Rotational Angiography. *AM J NEURORADIOL,* 01 April 2005, vol. 26, 908-911